# EUROPEAN PATENT APPLICATION

(11) **EP 2 400 024 A1**
(43) Date of publication of application: **28.12.2011**
(21) Application number: 10743768.3
(22) Date of filing: 17.02.2010
(51) Int. Cl.: C12P 1/04, C12N 15/09, C12N 1/21

(54) **PROCESS FOR PRODUCING USEFUL SUBSTANCE**

(30) Priority: 18.02.2009 JP 2009034692
(71) Applicant: Shinshu University, Matsumoto-shi, Nagano 390-8621 (JP); Kyowa Hakko Bio Co., Ltd., Tokyo 100-8185 (JP)
(72) Inventor: IKEDA, Masato, Kamiina-gun Nagano 399-4598 (JP); TAKENO, Seiki, Kamiina-gun Nagano 399-4598 (JP); MITSUHASHI, Satoshi, Tsukuba-shi Ibaraki 305-0841 (JP)
(74) Representative: Vossius & Partner
(86) International application number: PCT/JP2010/052341
(87) International publication number: WO 2010/095642

(57) **Abstract**

The present invention provides a process for producing a useful substance which comprises culturing a transformant obtained by transforming a microorganism capable of producing the useful substance with a DNA encoding a protein in a medium, wherein the protein has an activity to improve growth of a high concentration oxygen-requiring microorganism under low oxygen concentration; forming and accumulating the useful substance in a culture; and collecting the useful substance from the culture.

## Description

### Technical Field

The present invention relates to a process for producing a useful substance using a microorganism transformed with a DNA encoding a protein that has an activity to improve the growth of a high concentration oxygen-requiring microorganism under low oxygen concentration.

### BACKGROUND ART

Aerobic fermentation of a useful substance such as an amino acid, a nucleotide, etc., requires a large amount of oxygen. Insufficient oxygen causes a problem such that a toxic organic acid is accumulated in place of a desired product. For this reason, it is important to keep dissolved oxygen in a medium during culturing at more than a certain level. To overcome this problem, fermentation tanks having a high oxygen supply efficiency or oxygen enrichment membranes have been developed; however, the installation of such devices requires high costs. Therefore, a microorganism that does not require, during culturing, a large amount of dissolved oxygen is demanded.

Various genes that are assumed to be involved in oxygen utilization or oxygen stress resistance have been found on the genome of *Corynebacterium glutamicum.* For example, genes involved in a respiratory chain has been identified (see NPL 1). The *sigB* gene (see NPL 2) or *sigD* gene (see NPL1), which are the sigma factors of RNA polymerase, and the hemoglobin-like *glbo* gene (see PTL 2) have been found. Further, a group of genes (*nrdHIE* and *nrdF*) involved in ribonucleotide reductase has been identified (see NPL 3). Information of these genes are disclosed in the publication of a patent application (see PTL 3).

However, effects of enhanced expression of these genes on the dissolved oxygen level that a microorganism requires or on the amino acids productivity have not been known.

### Citation List

### Patent Literature

PTL 1: US Patent No. 6890744
PTL 2: US Patent No. 6759218
PTL 3: Japanese Unexamined Patent Publication No. 2002-191370

### Non-patent Literature

NPL 1: J. Biotechnol., 104, 129 (2003)
NPL 2: BMC Genomics, 8, 4 (2007)
NPL 3: Microbiology, 145, 1595 (1999)

### Summary of Invention

### Technical Problem

The present invention relates to a process for producing a useful substance using a microorganism transformed with a recombinant DNA that contains a DNA encoding a protein that has an activity to complement the high concentration oxygen-requiring properties of microorganism.

### Solution to Problem

The present invention provides the following items (1) to (3).
(1) A process for producing a useful substance which comprises culturing a transformant obtained by transforming a microorganism capable of producing the useful substance with a DNA encoding a protein in a medium, wherein the protein has an activity to improve growth of a high concentration oxygen-requiring microorganism under low oxygen concentration;
   forming and accumulating the useful substance in a culture; and collecting the useful substance from the culture.
(2) The process according to Item (1), wherein the protein having the activity to improve the growth of the high concentration oxygen-requiring microorganism under low oxygen concentration is a protein defined in any one of [1] to [6] below:
   [1] a protein comprising the amino acid sequence shown in SEQ ID NO: 1, 3, 5, 7, 9 or 11;
   [2] a protein consisting of the amino acid sequence, wherein one or more amino acid residues are deleted, substituted or added in the amino acid sequence shown in SEQ ID NO: 1, 3, 5, 7, 9 or 11, and having the activity to improve the growth of the high concentration oxygen-requiring microorganism under low oxygen concentration;
   [3] a protein consisting of the amino acid sequence which has 80% or more homology or identity to the amino acid sequence shown in SEQ ID NO: 1, 3, 5, 7, 9 or 11, and having the activity to improve the growth of the high concentration oxygen-requiring microorganism under low oxygen concentration;
   [4] a protein consisting of the amino acid sequence shown in SEQ ID NO: 13, 15, 17 or 19;
   [5] a protein consisting of the amino acid sequence, wherein one or more amino acid residues are deleted, substituted or added in the amino acid sequence shown in SEQ ID NO: 13, 15, 17 or 19, and having the activity to improve the growth of the high concentration oxygen-requiring microorganism under low oxygen concentration; and
   [6] a protein consisting of the amino acid sequence which has 80% or more homology or identity to the amino acid sequence shown in SEQ ID NO: 13, 15, 17 or 19, and that having the activity to improve the growth of the high concentration oxygen-requiring microorganism under low oxygen concentration. (3) The process according to Item (1) or (2), wherein the DNA encoding the protein having the activity to improve the growth of the high concentration oxygen-requiring microorganism under low oxygen concentration is a DNA defined in any one of [1] to [6] below:
      [1] DNA encoding the protein consisting of the amino acid sequence shown in SEQ ID NO: 1, 3, 5, 7, 9 or 11;
      [2] DNA comprising the nucleotide sequence shown in SEQ ID NO: 2, 4, 6, 8, 10 or 12;
      [3] DNA which hybridizes with a DNA consisting of the nucleotide sequence complementary to the nucleotide sequence shown in SEQ ID NO: 2, 4, 6, 8, 10 or 12 under stringent conditions, and codes for the protein having the activity to improve the growth of the high concentration oxygen-requiring microorganism under low oxygen concentration;
      [4] DNA encoding the protein consisting of the amino acid sequence shown in SEQ ID NO: 13, 15, 17 or 19;
      [5] DNA consisting of the nucleotide sequence shown in SEQ ID NO: 14, 16, 18 or 20; and
      [6] DNA which hybridizes with DNA consisting of the nucleotide sequence complementary to the nucleotide sequence shown in SEQ ID NO: 14, 16, 18 or 20 under stringent conditions, and encodes for the protein having the activity to improve the growth of the high concentration oxygen-requiring microorganism under low oxygen concentration.

### Advantageous Effects of Invention

According to the present invention, a process for producing a useful substance can be provided in which a transformant transformed with a DNA encoding a protein that has an activity to improve the growth of a high concentration oxygen-requiring microorganism under low oxygen concentration is used.

### Description of Embodiments

Any microorganism can be used as the transformant used in the production process of the present invention, insofar as the microorganism is a transformant obtained by transforming a microorganism having an ability to produce a useful substance with a DNA encoding a protein that has an activity to improve the growth of a high concentration oxygen-requiring microorganism under low oxygen concentration.

Examples of such microorganisms include those belonging to the genera *Corynebacterium, Brevibacterium, Microbacterium, Escherichia, Serratia, Bacillus, Pseudomonas* and *Streptomyces;* preferably those belonging to the genera Corynebacterium, *Brevibacterium* and *Microbacterizum;* and more preferably those belonging to the genus *Corynebacterium.*

Specific examples of microorganisms belonging to the genus Corynebacterium include *Corynebacterium acetoacidophilum* (e.g., *Corynebacteritum acetoacidophilum* ATCC13870), *Corynebacterium acetoglutamicum* (e.g., *Corynebacterium acetoacidophilum* ATCC15806), *Corynebacterium callunae* (e.g., *Corynebacterium callunae* ATCC15991), *Corynebacterium glutamicum* (e.g., *Corynebacterium glutamicum* ATCC13032, ATCC13060, ATCC13826, ATCC14020, and ATCC13869), *Corynebacterium herculis* (e.g., *Corynebacterium herculis* ATCC13868), *Corynebacterium lilium* (e.g., *Corynebacterium lilium* ATCC15990), *Corynebacterium melassecola* (e.g., *Corynebacterium melassecola* ATCC17965), *Corynebacterium thermoaminogenes* (e.g., *Corynebacterium thermoaminogenes* ATCC9244) and the like.

Specific examples of microorganisms belonging to the genus *Brevibacterium* include *Brevibacterium saccharolyticum* (e.g., *Brevibacterium saccharolyticum* ATCC14066), *Brevibacterium immariophilum* (e.g., *Brevibacterium immariophilum* ATCC14068), *Brevibacterium roseum* (e.g., *Brevibacterium roseum* ATCC13825), *Brevibacterium thiogenitalis* (e.g., *Brevibacterium thiogenitalis* ATCC19240) and the like.

Specific examples of microorganisms belonging to the genus *Microbacterium* include *Microbacterium ammoniaphilum* (e.g., *Microbacterium ammoniaphilum* ATCC15354) and the like.

The useful substance produced in the present invention can be any substance that can be produced by a microorganism belonging to coryneform bacteria and that is considered to be industrially useful. Examples of the useful substance include amino acids; nucleic acids; vitamins; proteins such as various enzymes; peptides such as glutathione; sugars such as xylose; sugar alcohols such as xylitol; alcohols such as ethanol; organic acids such as lactic acid and succinic acid; lipids; and the like. Amino acids, nucleic acids, and vitamins are preferable; and amino acids are particularly preferable.

Examples of amino acids include L-alanine, glycine, L-glutamine, L-glutamic acid, L-asparagine, L-aspartic acid, L-lysine, L-methionine, L-threonine, L-leucine, L-valine, L-isoleucine, L-proline, L-histidine, L-arginine, L-tyrosine, L-tryptophan, L-phenylalanine, L-serine, L-cysteine, L-3-hydroxyproline, L-4-hydroxyproline and the like. Examples of nucleic acids include inosine, guanosine, inosinic acid, guanylic acid, and the like. Examples of vitamins include riboflavin, thiamine, ascorbic acid and the like.

The "high concentration oxygen-requiring microorganism" in the present invention means a microorganism that is incapable of growing under low oxygen concentration (from 0.5% to atmospheric condition (about 21%), e.g., 0.5%, 1%, 3%, 6%, 9 etc.) in which a wild-type microorganism can grow, or a microorganism that is capable of growing under such conditions; however, the growth speed thereof is reduced to 50% or less, preferably 20% or less, and more preferably 10% or less compared with that of the wild-type strain.

The aforementioned low oxygen concentration can be set using an oxygen absorbent and a special hermetic container that are sold for simple cultivation of anaerobic bacterium or microaerophilic bacterium. In this method, an oxygen absorbent ("Anaero Pack series" produced by Mitsubishi Gas Chemical Co., Inc.), a plate medium and an aversion indicator are put and cultured in the hermetic container. The amount of oxygen absorbent can be adjusted according to the manual of the manufacturer to thereby formulate several different low oxygen concentrations.

In a medium for determining whether the microorganism is a high concentration oxygen-requiring microorganism, saccharides such as glucose, fructose, sucrose, maltose and starch hydrolyzate, alcohols such as ethanol, organic acids such as acetic acid, lactic acid and succinic acid can be used as a carbon source.

Examples of nitrogen sources include various inorganic and organic ammonium salts such as ammonia, ammonium chloride, ammonium sulfate, ammonium carbonate and ammonium acetate, urea and other nitrogen-containing compounds, nitrogen-containing organic substances such as meat extract, yeast extract, corn steep liquor and soybean hydrolysate and the like.

Examples of inorganic salts include dipotassium hydrogen phosphate, potassium dihydrogen phosphate, ammonium sulfate, sodium chloride, magnesium sulfate, calcium carbonate and the like.

If necessary, trace nutrient sources such as biotin, thiamine, etc., can also be added. Such trace nutrient sources can be substituted with a meat extract, yeast extract, corn steep liquor, soybean hydrolysate, casamino acid and other medium additives.

Whether the microorganism is a high concentration oxygen-requiring microorganism can be confirmed by culturing the microorganism at 20 to 42°C, preferably 30 to 40°C for 1 to 6 days under the aforementioned low oxygen concentration, and comparing the growth speed thereof with that of a wild-type strain.

Examples of the high concentration oxygen-requiring microorganism include *Corynebacterium glutamicum* strains OX-3, OX-96, OX-109, OX-112, OX-119 and OX-150.

In the present invention, the protein having an activity of improving the growth of a high concentration oxygen-requiring microorganism under low oxygen concentration indicates a protein that allows or improves the growth of the microorganism under low oxygen concentration when the high concentration oxygen-requiring microorganism is transformed by a DNA encoding the protein.

Examples of the protein include the following:
[1] a protein comprising the amino acid sequence shown in SEQ ID NO: 1, 3, 5, 7, 9 or 11;
[2] a protein consisting of the amino acid sequence, wherein one or more amino acid residues are deleted, substituted or added in the amino acid sequence shown in SEQ ID NO: 1, 3, 5, 7, 9 or 11, and having the activity to improve the growth of the high concentration oxygen-requiring microorganism under low oxygen concentration;
[3] a protein consisting of the amino acid sequence which has 80% or more homology or identity to the amino acid sequence shown in SEQ ID NO: 1, 3, 5, 7, 9 or 11, and having the activity to improve the growth of the high concentration oxygen-requiring microorganism under low oxygen concentration;
[4] a protein consisting of the amino acid sequence shown in SEQ ID NO: 13, 15, 17 or 19;
[5] a protein consisting of the amino acid sequence, wherein one or more amino acid residues are deleted, substituted or added in the amino acid sequence shown in SEQ ID NO: 13, 15, 17 or 19, and having the activity to improve the growth of the high concentration oxygen-requiring microorganism under low oxygen concentration; and
[6] a protein consisting of the amino acid sequence which has 80% or more homology or identity to the amino acid sequence shown in SEQ ID NO: 13, 15, 17 or 19, and having the activity to improve the growth of the high concentration oxygen-requiring microorganism under low oxygen concentration.

The proteins described in [1] to [6] can be expressed alone or in a combination of two or more to improve the growth under low oxygen concentration. For example, regarding the proteins described in [1] to [3], the proteins comprising the amino acid sequence shown in SEQ ID NOs: 1, 3, 5, 7, 9 and the variants thereof (proteins in which one or more amino acid residues are deleted, substituted or added, or proteins having 80% or more homology or identity) can be expressed alone or in a combination of two or more. Similarly, regarding the proteins described in [4] to [6], the proteins consisting of the amino acid sequence shown in SEQ ID NOs: 13, 15, 17, 19 and the variants thereof can be expressed alone or in a combination of two or more (e.g., three or more, or four) to improve the growth under low oxygen concentration.

The DNAs described in [1] to [6] can be expressed alone or in a combination of two or more to improve the growth under low oxygen concentration. For example, regarding the DNAs described in [1] to [3], DNAs comprising the nucleotide sequence shown in SEQ ID NOs: 2, 4, 6, 8, 10 and DNAs which hybridize with such DNAs under stringent conditions and codes for a protein having an activity to improve the growth under low oxygen concentration can be expressed alone; and the DNAs encoding protein can be expressed in a combination of two or more (e.g., three or more, or four).

The protein consisting of an amino acid sequence wherein one or more amino acid residues are deleted, substituted or added, and having an activity to improve the growth under low oxygen concentration of high concentration oxygen-requiring microorganisms, for example, coryneform bacteria, can be obtained, for example, by introducing a site-directed mutation into a DNA encoding a protein comprising the amino acid sequence shown in SEQ ID NO: 1, 3, 5, 7, 9, 11, 13, 15, 17 or 19 (for example, a DNA comprising the nucleotide sequence shown in SEQ ID NO: 2, 4, 6, 8, 10, 12, 14, 16, 18 or 20) by site-directed mutagenesis described in Molecular Cloning: A Laboratory Manual, Second Edition, Cold Spring Harbor Laboratory Press (1989) (hereinafter simply referred to as "Molecular Cloning, Second Edition"); Current Protocols in Molecular Biology, John Wiley & Sons (1987-1997) (hereinafter simply referred to as "Current Protocols in Molecular Biology"); Nucleic Acids Research, 10, 6487 (1982); Proc. Natl. Acad. Sci. USA, 79, 6409 (1982); Gene, 34, 315 (1985); Nucleic Acids Research, 13, 4431 (1985); Proc. Natl. Acad. Sci. USA, 82, 488 (1985); etc.

The number of amino acids that are deleted, substituted or added is not particularly limited, and is generally within the range in which deletion, substitution or addition can be done by a known method such as the above site-directed mutagenesis. The number of amino acids is typically 1 to several tens, preferably 1 to 20, more preferably 1 to 10 and still more preferably 1, 2, 3, 4 or 5.

The expression "an amino acid sequence, wherein one or more amino acid residues are added, deleted or substituted in the amino acid sequence shown in SEQ ID NO: 1, 3, 5, 7, 9, 11, 13, 15, 17 or 19" means that the amino acid sequence can include the deletion, substitution or addition of one or a plurality of amino acids at any position in the same sequence.

The position of an amino acid at which one or more amino acids can be deleted or added can be, for example, 1 to several amino acids from the N- or C-terminus of the amino acid sequence shown in SEQ ID NO: 1, 3, 5, 7, 9, 11, 13, 15, 17 or 19.

Deletion, substitution and addition can be simultaneously included in one sequence, and amino acids to be substituted or added can be natural or non-natural. Examples of the natural amino acids include L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, L-cysteine and the like.

Examples of the mutually substitutable amino acids are shown below. The amino acids in the same group are mutually substitutable.

Group A: leucine, isoleucine, norleucine, valine, norvaline, alanine, 2-aminobutanoic acid, methionine, O-methylserine, t-butylglycine, t-butylalanine and cyclohexylalanine.
Group B: aspartic acid, glutamic acid, isoaspartic acid, isoglutamic acid, 2-aminoadipic acid and 2-aminosuberic acid. Group C: asparagine and glutamine.
Group D: lysine, arginine, ornithine, 2,4-diaminobutanoic acid and 2,3-diaminopropionic acid.
Group E: proline, 3-hydroxyproline and 4-hydroxyproline.
Group F: serine, threonine and homoserine.
Group G: phenylalanine and tyrosine.

In the production process of the present invention, to impart the activity of improving the growth of the high concentration oxygen microorganism under low oxygen concentration to a protein, it is desirable that the protein of the present invention has 80% or more, preferably 90% or more, more preferably 95% or more, still more preferably 98% or more and particularly preferably 99% or more homology or identity to the amino acid sequence shown in SEQ ID NO: 1, 3, 5, 7, 9, 11, 13, 15, 17 or 19.

The homology or identity among amino acid sequences or nucleotide sequences can be determined by using algorithm BLAST by Karlin and Altschul (Pro. Natl. Acad. Sci. USA, 90, 5873 (1993)) and FASTA (Methods Enzymol., 183, 63 (1990)). Programs called BLASTN and BLASTX have been developed based on the algorithm BLAST (J. Mol. Biol. 215: 403 (1990)). For BLASTN analysis of a nucleotide sequence based on BLAST, the parameters are set, for example, to score = 100, and word length = 12. For BLASTX analysis of an amino acid sequence based on BLAST, the parameters are set, for example, to score = 50, and word length = 3. To obtain gapped alignments, Gapped BLAST can be utilized as described in Altschul et al. (1997, Nucleic Acids Res. 25: 3389-3402). Alternatively, PSI-Blast or PHI-Blast can be used to perform an iterated search that detects distant relationships between molecules (Id.), and relationships between molecules that share a common pattern. When utilizing BLAST, Gapped BLAST, PSI-Blast and PHI-Blast programs, the default parameters of the respective programs can be used. See http://www.ncbi.nlm.nih.gov.

Whether the protein has an activity of improving the growth of a microorganism under low oxygen concentration can be confirmed by checking whether the growth speed under low oxygen concentration of high concentration oxygen-requiring coryneform bacteria in which the protein is expressed is restored to 80% or more, preferably 90% or more compared with that of a wild-type strain.

Whether the proteins described in [4] to [6] have an activity to improve the growth under low oxygen concentration can be confirmed when the ribonucleotide reductase activity of the microorganism transformed with the DNAs encoding the proteins described in [4] to [6] is compared with that of a wild-type strain according to the method described in Microbiology, 145, 1595 (1999), and the microorganism has an improved ribonucleotide reductase activity compared to that of the wild-type strain.

Examples of the DNA that transforms the microorganism used in the production process of the present invention are DNAs following [7] to [12]:
[7] DNA encoding the protein consisting of the amino acid sequence shown in SEQ ID NO: 1, 3, 5, 7, 9 or 11;
[8] DNA comprising the nucleotide sequence shown in SEQ ID NO: 2, 4, 6, 8, 10 or 12;
[9] DNA which hybridizes with a DNA consisting of the nucleotide sequence complementary to the nucleotide sequence shown in SEQ ID NO: 2, 4, 6, 8, 10 or 12 under stringent conditions, and codes for the protein having the activity to improve the growth of the high concentration oxygen-requiring microorganism under low oxygen concentration;
[10] DNA encoding the protein consisting of the amino acid sequence shown in SEQ ID NO: 13, 15, 17 or 19;
[11] DNA consisting of the nucleotide sequence shown in SEQ ID NO: 14, 16, 18 or 20; and
[12] DNA which hybridizes with DNA consisting of the nucleotide sequence complementary to the nucleotide sequence shown in SEQ ID NO: 14, 16, 18 or 20 under stringent conditions, and encodes for the protein having the activity to improve the growth of a high concentration oxygen-requiring microorganism under low oxygen concentration.

The term "hybridize" in this specification indicates that a DNA hybridizes under specific conditions with another DNA having a specific nucleotide sequence or a portion of the DNA. Thus, the nucleotide sequence of the DNA having a specific nucleotide sequence or portion of the DNA can have a length such that the DNA or portion thereof can be used as a probe in Northern or Southern blot analysis or as oligonucleotide primers in PCR analysis. Examples of the DNA used as a probe include DNAs that are 100 nucleotides or more, preferably 200 nucleotides or more and more preferably 500 nucleotides or more in length. DNAs of 10 nucleotides or more, and preferably 15 nucleotides or more in length can also be used.

Methods for DNA hybridization experiments are well known. Such experiments can be conducted by determining hybridization conditions, for example, according to the disclosures in Molecular Cloning, Second and Third Editions (2001); Methods for General and Molecular Bacteriology, ASM Press (1994); Immunology Methods Manual, Academic Press (Molecular); and many other standard textbooks.

Hybridization under stringent conditions as mentioned above can be carried out, for example, in the following manner. A filter with DNA immobilized thereon and a probe DNA are incubated in a solution containing 50% formamide, 5 x SSC (750mM sodium chloride and 75mM sodium citrate), 50mM sodium phosphate (pH 7.6), 5 x Denhardt's solution, 10% dextran sulfate and 20µg/l denatured salmon sperm DNA at 42°C overnight, followed by washing the filter in 0.2 x SSC solution of about 65°C. Less-stringent conditions can also be employed. The stringent conditions can be modified by adjusting the concentration of formamide (the conditions become less stringent as the concentration of formamide is lowered) and by changing the salt concentration and the temperature conditions. Hybridization under less-stringent conditions can be carried out, for example, by incubating a filter with DNA immobilized thereon and a probe DNA in a solution containing 6 x SSCE (20 x SSCE: 3mol/l sodium chloride, 0.2mol/l sodium dihydrogenphosphate and 0.02mol/l EDTA, pH 7.4), 0.5% SDS, 30% formamide and 100µg/l denatured salmon sperm DNA at 37°C overnight, and washing the filter with 1 x SSC solution containing 0.1% SDS of 50°C. Hybridization under still less-stringent conditions can be carried out, for example, by performing hybridization using a solution having a high salt concentration (for example, 5 x SSC) under the above-mentioned less-stringent conditions, followed by washing.

The various conditions described above can also be set by adding or changing a blocking reagent to suppress the background in hybridization experiments. The addition of a blocking reagent as described above can also be accompanied by the alteration of hybridization conditions to make the conditions suitable for the purpose.

Examples of the DNA that hybridizes under stringent conditions include DNA having 80% or more, preferably 90% or more, more preferably 95% or more, still more preferably 98% or more and particularly preferably 99% or more homology or identity to the nucleotide sequence shown in SEQ ID NO: 2, 4, 6, 8, 10, 12, 14, 16, 18 or 20 as calculated by the use of programs such as BLAST or FASTA described above, based on the above parameters.

Whether a DNA that hybridizes with the above-mentioned DNA under stringent conditions is a DNA that encodes a protein having an activity to improve the growth of the microorganism under low oxygen concentration can be confirmed by checking whether the growth speed under low oxygen concentration of the transformant obtained by introducing the DNA into a high concentration oxygen-requiring microorganism, which is used as a host cell, is 80% or more, and preferably 90% or more compared with that of the wild-type strain.

The DNA that transforms the microorganism used in the production process of the present invention can be obtained from a microorganism belonging to coryneform bacteria by PCR using chromosomal DNA prepared by the method of Saito et al. (Biochim. Biophys. Acta, 72, 619 (1963)) as a template and using primer DNA designed and synthesized based on the nucleotide sequence shown in SEQ ID NO: 2, 4, 6, 8, 10, 12, 14, 16, 18 or 20.

The DNA of the present invention can be obtained, for example, by PCR using chromosomal DNA prepared from *Corynebacterium glutamicum* ATCC13032 or ATCC31833 as a template, and using DNA having 5'-terminal and 3'-terminal regions of the nucleotide sequence shown in SEQ ID NO: 2, 4, 6, 8, 10, 12, 14, 16, 18 or 20 as a primer set.

Specific examples of the DNA of the present invention that can be obtained include DNA comprising the nucleotide sequence shown in SEQ ID NO: 2, 4, 6, 8, 10, 12, 14, 16, 18 or 20.

The nucleotide sequences shown in SEQ ID NOs: 2, 4, 6, 8, 10, 12, 14, 16, 18 and 20 are disclosed as Cgl0807, Cgl0600, Cgl1427, Cgl1102, Cgl2859, Cgl2857, Cgl2525, Cgl2530, Cgl2531 and Cgl2532, respectively, in the nucleotide sequence represented by NCBI GenBank accession No. BA000036.

Cgl0807, Cgl0600, Cgl1427, Cgl1102, Cgl2859, Cgl2857, Cgl2525, Cgl2530, Cgl2531 and Cgl2532 respectively correspond to NCgl0773, NCgl0575, NCgl1372, NCgl1057, NCgl2761, NCgl2760, NCgl2438, NCgl2443, NCgl2444 and NCgl2445 in the nucleotide sequence shown in GenBank accession No. NC_003450.

The DNA of the present invention can also be obtained by a hybridization method using partial or full-length DNA consisting of the nucleotide sequence shown in SEQ ID NO: 2, 4, 6, 8, 10, 12, 14, 16, 18 or 20 as a probe; or by a known method such as chemical synthesis based on the nucleotide sequence shown in SEQ ID NO: 2, 4, 6, 8, 10, 12, 14, 16, 18 or 20.

The DNA of the present invention or DNA used in the production process of the present invention can also be obtained by conducting a search through various gene sequence databases for a sequence having 85% or more, preferably 90% or more, more preferably 95% or more, still more preferably 98% or more and particularly preferably 99% or more homology or identity to a nucleotide sequence of DNA encoding the amino acid sequence shown in SEQ ID NO: 1, 3, 5, 7, 9, 11, 13, 15, 17, or 19; and obtaining the desired DNA based on the nucleotide sequence obtained by the search from a chromosomal DNA or cDNA library of an organism having the nucleotide sequence according to the above-described method.

The nucleotide sequence of the DNA can be determined by a conventional sequencing method such as the dideoxy method (Proc. Natl. Acad. Sci., USA, 74, 5463 (1977)), or by using a nucleotide sequencer such as a 373A DNA Sequencer (a product of Perkin-Elmer Corp.).

In cases where the obtained DNA is found to be a partial DNA by the nucleotide sequence analysis, the full-length DNA can be obtained by Southern hybridization for a chromosomal DNA library using the partial DNA as a probe.

The obtained DNA can be used as-is. If necessary, the DNA can be modified by substituting one or more nucleotides to obtain the codon that is most suitable for the expression of coryneform bacteria. Further, if necessary, a DNA fragment having a suitable length and containing a region that encodes the protein of the present invention can be prepared. The DNA or DNA fragment is ligated downstream of a promoter of an expression vector suitable for the expression of coryneform bacteria to produce a recombinant DNA.

The vectors that can be preferably used are those capable of autonomous replication or integration into the chromosome in a microorganism and containing a promoter at a position appropriate for the transcription of DNA to be transformed.

When a prokaryotic organism such as bacteria is used as a host cell, it is preferable that the recombinant DNA having the DNA of the present invention is capable of autonomous replication in a prokaryotic organism and comprise a promoter, a ribosome binding sequence, the DNA of the present invention and a transcription termination sequence. A gene that controls a promoter can also be contained in the recombinant DNA.

When coryneform bacteria is used as a host cell, pCG1 (Japanese Unexamined Patent Publication No. S57-134500); pCG2 (Japanese Unexamined Patent Publication No. S58-35197); pCG4 (Japanese Unexamined Patent Publication No. S57-183799); pCG11 (Japanese Unexamined Patent Publication No. S57-134500); pCG116, pCE54 and pCB101 (all disclosed in Japanese Unexamined Patent Publication No. S58-105999); pCE51, pCE52 and pCE53 (all described in Molecular and General Genetics, 196, 175 (1984)); and the like are preferably used.

The recombinant DNA produced by inserting the DNA of the present invention into a vector preferably comprises a promoter, a ribosome binding sequence, the DNA of the present invention and a transcription termination sequence. A gene that controls a promoter can also be contained in the recombinant DNA.

Any promoter that functions in host cells (coryneform bacteria) can be used. Examples of promoters include promoters derived from *E. coli* or phages such as *trp* promoter (P*ₜᵣₚ*), *lac* promoter, P_{L} promoter, P_{R} promoter and T7 promoter. Artificially designed or modified promoters can also be used, and examples thereof include a promoter wherein two Pₜᵣₚ are tandemly arranged (Pₜᵣₚ x 2), tac promoter, T7-lac promoter, let I promoter and P54-6 promoter for expression in microorganisms belonging to the genus *Corynebacterium* (Appl. Microbiol. Biotechnol., 53, 674-679 (2000)).

It is preferable that the distance between the Shine-Dalgarno sequence, which is a ribosome-binding sequence, and the initiation codon is adjusted appropriately (for example, 6 to 18 nucleotides). Although a transcription termination sequence is not always necessary, it is preferable to place a transcription termination sequence immediately downstream of the structural gene.

As a method for introducing the recombinant DNA, any method capable of introducing DNA into bacteria can be used. Examples of the methods include the method using calcium ions (Proc. Natl. Acad. Sci., USA, 69, 2110 (1972)), the protoplast method (Japanese Unexamined Patent Publication No. 1988-248394), the electroporation method (Nucleic Acids, Res., 16, 6127 (1988)) and the like.

The DNA encoding a protein having an activity to improve the growth of high concentration oxygen-requiring microorganism under low oxygen concentration can be integrated into a chromosome of coryneform bacteria, or can be introduced as a plasmid into coryneform bacteria to form a transformant. The DNA that has an activity to improve the growth of high concentration oxygen-requiring microorganism under low oxygen concentration is preferably derived from coryneform bacteria.

When a microorganism used as a host cell has an ability to produce a useful substance such as an amino acid, a nucleic acid, etc., the obtained transformant can be used in the production of the useful substance without any treatment. However, when a microorganism does not have such an ability, a useful substance can be obtained by breeding a wild-type strain according to known methods.
Examples of the known methods include the following:
(a) the method of reducing or removing at least one mechanism of controlling the biosynthesis of a useful substance;
(b) the method of enhancing the expression of at least one enzyme involved in the biosynthesis of a useful substance;
(c) the method of increasing the copy number of at least one enzyme gene involved in the biosynthesis of a useful substance;
(d) the method of attenuating or blocking at least one metabolic pathway to produce metabolites other than a useful substance, which branches off from a biosynthetic pathway of the useful substance; and
(e) the method of selecting a cell strain that is highly resistant to an analogue of a useful substance, as compared with a wild-type strain.
The above known methods can be used alone, or in a combination of two or more.

Specific examples of the above methods (a) to (e), for example, to produce an amino acid as a useful substance are described in the following publications. Method (a) is described in Agric. Biol. Chem., 43, 105-111 (1979); J. Bacteriol., 110, 761-763 (1972); Appl. Microbiol. Biotechnol., 39, 318-323 (1993); and the like. Method (b) is described in Agric. Biol. Chem., 43, 105-111 (1979); J. Bacteriol., 110, 761-763 (1972); and the like. Method (c) is described in Appl, Microbiol. Biotechnol., 39, 318-323 (1993); Agric. Biol. Chem., 39, 371-377 (1987); and the like. Method (d) is described in Appl. Environ. Microbiol., 38, 181-190 (1979); Agric. Biol. Chem., 42, 1773-1778 (1978); and the like. Method (e) is described in Agric. Biol. Chem., 36, 1675-1684 (1972); Agric. Biol. Chem., 41, 109-116 (1977); Agric. Biol. Chem., 37, 2013-2023 (1973); Agric. Biol. Chem., 51, 2089-2094 (1987); and the like. As reference to these publications, microorganisms capable of forming and accumulating various amino acids can be bred and obtained.

Further, many methods for breeding a microorganism capable of forming an amino acid according to any one of, or a combination of the above methods (a) to (e) are described in Biotechnology 2nd ed., vol. 6, Products of Primary Metabolism (VCH Verlagsgesellschaft mbH, Weinheim, 1996), section 14a or 14b; Advances in Biochemical Engineering/Biotechnology 79, 1-35 (2003); and Amino san Hakko (Amino acid fermentation), Japan Scientific Societies Press, Hiroshi Aida et al. (1986). Specific methods for breeding a microorganism capable of forming and accumulating an amino acid are also described in many reports other than the above-mentioned publications, such as Japanese Unexamined Patent Publication No. 2003-164297; Agric. Biol. Chem., 39, 153-160 (1975); Agric. Biol. Chem., 39, 1149-1153 (1975); Japanese Unexamined Patent Publication No. 1983-13599; J. Gen. Appl. Microbiol., 4, 272-283, (1958); Japanese Unexamined Patent Publication No. 1988-94985; Agric. Biol. Chem., 37, 2013-2023 (1973); WO 97/15673; Japanese Unexamined Patent Publication No. 1981-18596; Japanese Unexamined Patent Publication No. 1981-144092; and Japanese Unexamined Patent Publication No. 2003-511086. As reference to these publications, a microorganism having an ability to produce an amino acid can be bred.

The useful substance can be obtained by collecting the useful substance produced and accumulated in a culture obtained by culturing a transformant in a medium using the production process of the present invention.

The transformant used in the production process of the present invention is cultured according to an ordinal method for culturing a microorganism. It is preferable to use the most appropriate conditions depending on a useful substance to be produced.

The carbon sources, nitrogen sources, inorganic salts and other additives of the medium for culturing the transformant are the same as those used in the medium for determining the high concentration oxygen requirement.

Culturing of the transformant is performed under aerobic conditions by a shaking culture, deep aeration agitation culture or the like. In general, the culture temperature is from 20 to 42°C and preferably from 30 to 40°C. The pH of the medium is preferably maintained in the range of pH 5 to pH 9. The pH of the medium is adjusted by using an inorganic or organic acid, alkaline solution, urea, calcium carbonate, ammonia, etc.

The culturing period generally ranges from 1 to 6 days. During cultivation, antibiotics such as ampicillin and tetracycline may be added to the medium, if necessary.

When the microorganism transformed with a recombinant DNA using an inductive promoter as a promoter is cultured, an inducer may be added to the medium, if necessary. For example, when the microorganism transformed with the recombinant vector comprising *lac* promoter is cultured, isopropyl-β-D-thiogalactopyranoside, etc. may be added to the medium; when the microorganism transformed with the recombinant vector comprising a *trp* promoter is cultured, indole acrylic acid etc. may be added to the medium.

Regarding the useful substance produced and accumulated in the culture, precipitates such as cells are removed from the culture, and then the target useful substance can be collected from the culture by using a combination of known methods such as activated carbon treatment and ion exchange resin treatment.

### EXAMPLES

The examples below serve to illustrate the present invention, but should not be construed as limitations on the scope of the invention.

### [Example 1]

(1) The *Corynebacterium glutamicum* strain ATCC31833 (hereinafter referred to as ATCC31833 strain) was mutagenized with nitrosoguanidine according to a usual method. The ATCC31833 strain treated with the mutagen was spread on BY agar medium (20g of normal bouillon, 5g of yeast extract and 12g of Ina agar S-7 in 1 L of water; and adjusted to pH 7.2 by an aqueous sodium hydroxide solution), and then cultured under atmospheric condition (oxygen concentration: 21%) and 0.5% oxygen concentration. The oxygen concentration was set using an Anaero Pack Kenki (produced by Mitsubishi Gas Chemical Co., Inc.), according to the manual therefor. Using the replica method, strains that did not grow well under 0.5% oxygen concentration compared to the strains under atmospheric condition, or strains that could not grow under 0.5% oxygen concentration were selected as high concentration oxygen-requiring strains. From the selected strains, six kinds of variants were selected as representative strains, and they were named as OX-3, OX-96, OX-109, OX-112, OX-119 and OX-150, respectively. The strains were cultured on BY agar medium for 2 days at 30°C under the various oxygen concentrations shown in Table 1. Table 1 shows the results of the growth of six strains under different oxygen concentrations. In Table 1, "++" indicates good growth, "+" indicates bad growth, and "-" indicates no growth.

**[Table 1]**

| Growth under each oxygen concentration | | | | | |
|---|---|---|---|---|---|
| Strains | Oxygen concentration | | | | |
| | 21% | 9% | 6% | 3% | 0.5% |
| ATCC31833 | ++ | ++ | ++ | ++ | + |
| OX-3 | ++ | + | - | - | - |
| OX-96 | + | + | - | - | - |
| OX-109 | + | + | - | - | - |
| OX-112 | + | + | - | - | - |
| OX-119 | + | + | - | - | - |
| OX-150 | ++ | ++ | ++ | + | - |

As shown in Table 1, the strains OX-3, OX-96, OX-109, OX-112, OX-119 and OX-150 showed high concentration oxygen-requiring properties as compared to the parent strain ATCC31833.

(2) The chromosomal DNA of ATCC31833 strain was prepared according to the method described in Japanese Unexamined Patent Publication No. H6-169785.

The chromosomal DNA of ATCC31833 strain and plasmid pCSEK20 (WO01/02177 were individually digested with EcoRI, and the fragments were ligated with a ligation kit. The chromosomal DNA of ATCC31833 strain and plasmid pCS299P (Appl. Microbiol. Biotech., 63, 592 (2004)) were individually digested with BamHI, and then the fragments were ligated with a ligation kit.

The high concentration oxygen-requiring strains obtained in (1), i.e., OX-3, OX-96, OX-109, OX-112, OX-119 and OX-150 were transformed with the thus-prepared two kinds of genomic libraries, according to the method described in Japanese Unexamined Patent Publication No. H6-169785.

From the obtained transformants, strains that were capable of growing under 0.5% oxygen concentration in BY agar medium containing 20µg/mL of kanamycin were selected as transformants in which high concentration oxygen-requiring properties were restored. According to the vector preparation method described in Japanese Unexamined Patent Publication No. H6-169785, plasmid DNA was isolated from each of the selected transformants.

Each of the obtained various plasmid DNAs was digested and analyzed with restriction enzymes. As a result, 1.9Kb EcoRI cleavage fragment was obtained as a DNA fragment for restoring the high concentration oxygen-requiring properties of OX-3, 2.1Kb BamHI cleavage fragment as a DNA fragment for restoring the high concentration oxygen-requiring properties of OX-96, 3.2Kb EcoRI cleavage fragment as a DNA fragment for restoring the high concentration oxygen-requiring properties of OX-109, 1.1Kb EcoRI cleavage fragment as a DNA fragment for restoring the high concentration oxygen-requiring properties of OX-112, 1.8Kb BamHI cleavage fragment and 3.2Kb BamHI cleavage fragment as DNA fragments for restoring the high concentration oxygen-requiring properties of OX-119, and 4.4Kb EcoRI cleavage fragment as a DNA fragment for restoring the high concentration oxygen-requiring properties of OX-150. The thus-obtained plasmid containing 1.9Kb EcoRI fragment, plasmid containing 2.1Kb BamHI fragment, plasmid containing 3.2Kb EcoRI fragment, plasmid containing 1.lKb EcoRI fragment, plasmid containing 1.8Kb BamHI fragment, plasmid containing 3.2Kb BamHI fragment and plasmid containing 4.4Kb EcoRI fragment were named as pEco1.9, pBam2.1, pEco3.2, pEco1.1, pBam1.8 pBam3.2 and pEco4.4, respectively.

The nucleotide sequence of each DNA fragment inserted in pEco1.9, pBam2.1, pEco3.2, pEco1.1, pBam1.8, pBam3.2 and pEco4.4 was determined according to a known method.

As a result of the analysis of the determined nucleotide sequences based on the entire genome information of the *Corynebacterium glutamicum* (NCBI GenBank Accession No. BA000036), Cgl0807 was present in the 1.9Kb EcoRI fragment contained in the pEco1.9. Cgl1101 and Cgl1102 were present in the 2.1Kb BamHI fragment contained in the pBam2.1. Cgl2859 and Cgl2861 were present in the 3.2Kb EcoRI fragment contained in the pEco3.2. Cgl0600 was present in the 1.1Kb EcoRI fragment contained in the pEco1.1. Cgl1427 was present in the 1.8Kb BamHI fragment contained in the pram1.8. Cgl2857 and Cgl2858 were present in the 3.2Kb BamHI fragment contained in the pBam3.2. Four genes, i.e., Cgl2530, Cgl2531, Cgl2532 and Cgl2533, were present in the 4.4Kb EcoRI fragment contained in pEco4.4.

The amino acid sequence inferred from the nucleotide sequence of the Cgl0807 gene that complements the high concentration oxygen-requiring properties of OX-3 strain is shown in SEQ ID NO: 1, and the nucleotide sequence is shown in SEQ ID NO: 2.

The amino acid sequence inferred from the nucleotide sequence of the Cgl0600 gene that satisfies the high concentration oxygen-requiring properties of OX-112 strain is shown in SEQ ID NO: 3, and the nucleotide sequence is shown in SEQ ID NO: 4.

The amino acid sequence inferred from the nucleotide sequence of the Cgl1427 gene that satisfies the high concentration oxygen-requiring properties of OX-119 strain is shown in SEQ ID NO: 5, and the nucleotide sequence is shown in SEQ ID NO: 6.

The ability of restoring the high concentration oxygen-requiring properties of OX-96 strain was examined by subcloning various DNA fragments from pBam2.1 according to a usual method. The results showed that the high concentration oxygen-requiring properties of OX-96 strain were restored by the plasmid pCgl1102 that contains a Cgl1102 gene alone. Plasmid pCgl1102 was obtained by treating a 0.5Kb DNA fragment and vector pCS299P with BamHI restriction enzyme, wherein the 0.5Kb of DNA fragment was obtained by PCR using plasmid pBam2.1 as a template and DNAs consisting of the nucleotide sequences shown in SEQ ID NOs: 21 and 22 as primers, and then ligating them to each other. The amino acid sequence inferred from the nucleotide sequence of Cgl1102 gene is shown in SEQ ID NO: 7, and the nucleotide sequence is shown in SEQ ID NO: 8.

Similarly, the ability of restoring the high concentration oxygen-requiring properties of OX-109 strain was also examined by subcloning various DNA fragments from pEco3.2 according to a usual method. As a result, the high concentration oxygen-requiring properties of OX-109 strain was restored by the plasmid pCgl2859 containing a Cgl2859 gene alone. Plasmid pCgl2859 was obtained by digesting with BamHI and removing Cgl2861 from plasmid pEco3.2 and ligating them again. The amino acid sequence inferred from the nucleotide sequence of Cgl2859 gene is shown in SEQ ID NO: 9, and the nucleotide sequence is shown in SEQ ID NO: 10.

The ability of restoring the high concentration oxygen-requiring properties of OX-119 strain was also examined by subcloning various DNA fragments from pBam3.2 according to a usual method. As a result, the high concentration oxygen-requiring OX-119 strain was restored by the plasmid pCgl2857 containing a Cgl2857 gene alone. Plasmid pCgl2857 was obtained by treating a 1.4Kb DNA fragment and vector pCS299P with BamHI restriction enzyme, wherein the 1.4Kb DNA fragment was obtained by PCR using plasmid pBam3.2 as a template and DNAs consisting of the nucleotide sequences shown in SEQ ID NOs: 23 and 24 as primers, and then ligating them to each other. The amino acid sequence inferred from the nucleotide sequence of Cgl2857 gene is shown in SEQ ID NO: 11, and the nucleotide sequence is shown in SEQ ID NO: 12.

The ability of restoring the high concentration oxygen-requiring properties of OX-150 strain was also examined by subcloning various DNA fragments from pEco4.4 according to a usual method. As a result, the high concentration oxygen-requiring properties of OX-150 strain were restored by the plasmid pHIE containing genes Cgl2530, Cgl2531 and CGl2532. Plasmid pHIE was obtained by treating a 3.8Kb DNA fragment and vector pCS299P with KpnI restriction enzyme, wherein the 3.8Kb DNA fragment was obtained by PCR using chromosomal DNA of ATCC31833 strain as a template and DNAs consisting of the nucleotide sequences shown in SEQ ID NOs: 25 and 26 as primers, and then ligating them to each other.

It is reported that these three genes are ribonucleotide reductase-related proteins, and that Cgl2525 is a constituting factor of the enzyme (Microbiology, 145, and 1595 (1999)). The plasmid pHIEF containing these 4 genes was constructed, and the complementation of the high concentration oxygen-requiring properties of OX-150 strain was examined. As a result, the plasmid pHIEF containing all 4 genes showed higher complementation ability than the plasmid pHIE containing only 3 genes. pHIEF was obtained by treating a 1.6Kb DNA fragment and vector pCS299P with BamHI restriction enzyme, wherein the 1.6Kb DNA fragment was obtained by PCR using chromosomal DNA of ATCC31833 strain as a template and DNAs consisting of the nucleotide sequences shown in SEQ ID NOs: 27 and 28 as primers, and then ligating them to each other.

The amino acid sequence inferred from the nucleotide sequence of Cgl2525 gene is shown in SEQ ID NO: 13, and the nucleotide sequence is shown in SEQ ID NO: 14. The amino acid sequence inferred from the nucleotide sequence of Cgl2530 gene is shown in SEQ ID NO: 15, and the nucleotide sequence is shown in SEQ ID NO: 16. The amino acid sequence inferred from the nucleotide sequence of Cgl2531 gene is shown in SEQ ID NO: 17, and the nucleotide sequence is shown in SEQ ID NO: 18. The amino acid sequence inferred from the nucleotide sequence of Cg12532 gene is shown in SEQ ID NO: 19, and the nucleotide sequence is shown in SEQ ID NO: 20.

(3) The following strains were cultured in BY agar medium for 2 days at 30°C under the different oxygen concentrations shown in Table 2: OX-3 strain, OX-3/pEco1.9 strain in which plasmid pEco1.9 was introduced in OX-3 strain; OX-96 strain, OX-96/pCgl1102 strain in which plasmid pCgl1102 was introduced in OX-96 strain; OX-109 strain, OX-109/pCgl2859 strain in which plasmid pCgl2859 was introduced in OX-109 strain; OX-112 strain, OX-112/pEcol.1 strain in which plasmid pEcol.1 was introduced in OX-112 strain; OX-119 strain, OX-119/pBam1.8 strain in which plasmid pBaml.8 was introduced in OX-119 strain; OX-119/pCg12857 strain in which plasmid pCgl2857 was introduced in OX-119 strain; and OX-150 strain, and OX-150/pHIEF strain in which plasmid pHIEF was introduced in OX-150. Table 2 shows the results. In Table 2, "++" indicates good growth, "+" indicates bad growth, and "-" indicates no growth.

**[Table 2] Growth under each oxygen concentration**

| Strains | Oxygen concentration | | | | |
|---|---|---|---|---|---|
| | 21% | 9% | 6% | 3% | 0.5% |
| ATCC31833 | ++ | ++ | ++ | ++ | + |
| OX-3 | ++ | + | - | - | - |
| OX-3/pEco1.9 | ++ | ++ | + | + | + |
| OX-96 | + | + | - | - | - |
| OX-96/pCgl1102 | ++ | ++ | + | + | + |
| OX-109 | + | + | - | - | - |
| OX-109/pCgl2859 | ++ | ++ | ++ | + | + |
| OX-112 | + | + | - | - | - |
| OX-112/pEco1.1 | ++ | ++ | + | + | + |
| OX-119 | + | + | - | - | - |
| OX-119/pBam1.8 | ++ | ++ | + | - | - |
| OX-119/pCgl2857 | ++ | ++ | ++ | + | + |
| OX-150 | ++ | ++ | ++ | + | - |
| OX-150/pHIEF | ++ | ++ | ++ | ++ | + |

As shown in Table 2, in the OX-3/pEco1.9 strain, OX-96/pCgl1102 strain, OX-109/pCgl2859 strain, OX-112/pEco1.1 strain, OX-119/pBam1.8 strain, OX-119/pCgl2857 strain and OX-150/pHIEF strain, high concentration oxygen-requiring properties of those strains were restored compared to the each host strains.

### Example 2

An L-lysine- producing bacterium was transformed with plasmid pEco1.9 pCgl1102, pCgl2859, pEco1.1, pBam1.8, pCgl2857 and pHIEF obtained in Example 1, according to the method described in Japanese Unexamined Patent Publication No. H6-169785. As the L-lysine-producing bacterium, a *Corynebacterium glutamicum* AHP-3 strain (FERM BP-7382), whose genetic character was known, was used. The *Corynebacterium glutamicum* AHP-3 strain has Val59Ala amino acid substitution in the homoserine dehydrogenase gene (hom), Thr331IIe amino acid substitution in the aspartokinase gene (lysC), and Pro458Ser amino acid substitution in the pyruvate carboxylase gene (pyc) on the chromosome of wild strain ATCC13032 of the *Corynebacterium glutamicum.* According to the preparation method of a vector disclosed in Japanese Unexamined Patent Publication No. 1994-169785, plasmid DNA was isolated from each of the transformed strains having kanamycin resistance. It was confirmed that each transformed strain had each plasmid by the cleavage analysis of the plasmids using various restriction enzymes.

The L-lysine production tests of these transformed strains and parent strains were conducted as follows. One platinum loop amount of cells which was cultured at 30°C for 24 hours on the BY agar medium (7g of meat extract, 10g of peptone, 3g of sodium chloride, 5g of yeast extract, and 15g of Bacto agar in 1L of water; and adjusted to pH 7.2), was inoculated into a large test tube containing 5mL of seed medium (20g of glucose, 7g of meat extract, 10g of peptone, 3g of sodium chloride, and 5g of yeast extract in 1L of water; and adjusted to pH 7.2, after which 10g of calcium carbonate was added), and cultured at 30°C for 13 hours. 0.5mL of this seed culture solution was inoculated into a large test tube containing 7mL of main culture medium (50g of glucose, 10g of corn steep liquor, 45 g of ammonium sulfate, 2g of urea, 0.5g of potassium dihydrogen phosphate, 0.5g of magnesium sulfate heptahydrate, and 0.3mg of biotin in 1L of water; and adjusted to pH 7.0, after which 30g of calcium carbonate was added), and cultured on a shaker at 30°C for 72 hours. After the cells were removed from the culture by centrifugation, the amount of L-lysine monohydrochloride accumulated in the supernatant was determined by high-performance liquid chromatography (HPLC). Table 3 shows the results.

**[Table 3]**

| Test of L-lysine Production | | |
|---|---|---|
| Strains | Growth (OD₆₆₀) | L-lysine hydrochloride (g/l) |
| AHP-3 | 15 | 4.4 |
| AHP-3/pEcol.9 | 16 | 5.0 |
| AHP-3/pCgl1102 | 16 | 5.0 |
| AHP-3/pCgl2859 | 17 | 5.2 |
| AHP-3/pEco1.1 | 16 | 5.1 |
| AHP-3/pBam1.8 | 18 | 5.2 |
| AHP-3/pCgl2857 | 17 | 5.2 |
| AHP-3/pHIEF | 15 | 5.0 |

As is clear from Table 3, the AHP-3 strain having the plasmid containing the gene of the present invention produced L-lysine in a significantly increased amount, as compared to the parent strain AHP-3.

### [Industrial Applicability]

According to the present invention, a process for producing a useful substance using a transformant transformed with a DNA encoding a protein having an activity to improve the growth of a high concentration oxygen-requiring microorganism under low oxygen concentration can be provided.

SEQ ID NO: 21 - Description of Artificial Sequence: Synthetic DNA
SEQ ID NO: 22 - Description of Artificial Sequence: Synthetic DNA
SEQ ID NO: 23 - Description of Artificial Sequence: Synthetic DNA
SEQ ID NO: 24 - Description of Artificial Sequence: Synthetic DNA
SEQ ID NO: 25 - Description of Artificial Sequence: Synthetic DNA
SEQ ID NO: 26 - Description of Artificial Sequence: Synthetic DNA
SEQ ID NO: 27 - Description of Artificial Sequence: Synthetic DNA
SEQ ID NO: 28 - Description of Artificial Sequence: Synthetic DNA

## Claims

1. A process for producing a useful substance which comprises culturing a transformant obtained by transforming a microorganism capable of producing the useful substance with a DNA encoding a protein in a medium, wherein the protein has an activity to improve growth of a high concentration oxygen-requiring microorganism under low oxygen concentration;
forming and accumulating the useful substance in a culture; and collecting the useful substance from the culture.

2. The process according to claim 1, wherein the protein having the activity to improve the growth of the high concentration oxygen-requiring microorganism under low oxygen concentration is a protein defined in any one of [1] to [6] below:
[1] a protein comprising the amino acid sequence shown in SEQ ID NO: 1, 3, 5, 7, 9 or 11;
[2] a protein consisting of the amino acid sequence, wherein one or more amino acid residues are deleted, substituted or added in the amino acid sequence shown in SEQ ID NO: 1, 3, 5, 7, 9 or 11, and having the activity to improve the growth of the high concentration oxygen-requiring microorganism under low oxygen concentration;
[3] a protein consisting of the amino acid sequence which has 80% or more homology or identity to the amino acid sequence shown in SEQ ID NO: 1, 3, 5, 7, 9 or 11, and having the activity to improve the growth of the high concentration oxygen-requiring microorganism under low oxygen concentration;
[4] a protein consisting of the amino acid sequence shown in SEQ ID NO: 13, 15, 17 or 19;
[5] a protein consisting of the amino acid sequence, wherein one or more amino acid residues are deleted, substituted or added in the amino acid sequence shown in SEQ ID NO: 13, 15, 17 or 19, and having the activity to improve the growth of the high concentration oxygen-requiring microorganism under low oxygen concentration; and
[6] a protein consisting of the amino acid sequence which has 80% or more homology or identity to the amino acid sequence shown in SEQ ID NO: 13, 15, 17 or 19, and having the activity to improve the growth of the high concentration oxygen-requiring microorganism under low oxygen concentration.

3. The process according to claim 1 or 2, wherein the DNA encoding the protein having the activity to improve the growth of the high concentration oxygen-requiring microorganism under low oxygen concentration is a DNA defined in any one of [1] to [6] below:
[1] DNA encoding the protein consisting of the amino acid sequence shown in SEQ ID NO: 1, 3, 5, 7, 9 or 11;
[2] DNA comprising the nucleotide sequence shown in SEQ ID NO: 2, 4, 6, 8, 10 or 12;
[3] DNA which hybridizes with a DNA consisting of the nucleotide sequence complementary to the nucleotide sequence shown in SEQ ID NO: 2, 4, 6, 8, 10 or 12 under stringent conditions, and codes for the protein having the activity to improve the growth of the high concentration oxygen-requiring microorganism under low oxygen concentration;
[4] DNA encoding the protein consisting of the amino acid sequence shown in SEQ ID NO: 13, 15, 17 or 19;
[5] DNA consisting of the nucleotide sequence shown in SEQ ID NO: 14, 16, 18 or 20; and
[6] DNA which hybridizes with DNA consisting of the nucleotide sequence complementary to the nucleotide sequence shown in SEQ ID NO: 14, 16, 18 or 20 under stringent conditions, and encodes for the protein having the activity to improve the growth of the high concentration oxygen-requiring microorganism under low oxygen concentration.


SEQUENCE LISTING

<110>  SHINSHU UNIVERSITY
<110>  KYOWA HAKKO BIO CO., LTD.

<120>  Process for producing useful substance

<130>  T2617 EP S3

<140>  EP 10 74 3768.3
<141>  2010-02-17

<150>  JP 2009-034692
<151>  2009-02-18

<160>  28

<170>  PatentIn version 3.4

<210>  1
<211>  275
<212>  PRT
<213>  Corynebacterium glutamicum 0807

<400>  1

Met Ser Thr Ala Leu Pro Asp Gln Leu Lys Trp Glu Tyr Ser Ala Phe
1               5                   10                  15

Pro Val Gln Ile Ser Gln Lys Gln Arg Leu Ser Pro Gly Phe Met Arg
            20                  25                  30

Ile Thr Val Thr Gly Asp Lys Leu Arg Phe Phe Gly Gln Trp Gly Leu
        35                  40                  45

Asp Gln Arg Ile Lys Leu Ile Ile Pro Ser Pro Ala Gly Asn Ile Pro
    50                  55                  60

Asp Phe Gly Ile Leu Asp Glu Pro Thr Pro Pro Pro Thr Thr Trp Leu
65                  70                  75                  80

Pro Arg Ala Lys Ser Phe Pro Ala Asp Gln Arg Pro Ile Leu Arg Thr
                85                  90                  95

Tyr Thr Pro Ser Ala Val Arg Pro Glu Leu Cys Glu Val Asp Ile Asp
            100                 105                 110

Ile Tyr Leu His Asn Pro Ser Gly Pro Val Ser Arg Trp Ala Lys Asn
        115                 120                 125

Cys Ser Val Asp Asp Glu Leu Ile Ile Thr Gly Pro Asp Val Arg Ala
    130                 135                 140

Gly Glu Thr Gly Tyr Gly Ile Thr Tyr His Pro Thr Ser Ala Ile Asp
145                 150                 155                 160

Arg Leu Cys Leu Ile Gly Asp Cys Ala Ser Ala Pro Ala Ile Ala Asn
                165                 170                 175

Ile Val Asn Gln Ser Lys Val Pro Thr Thr Val Phe Leu His Val Asp
            180                 185                 190

Ser Leu Glu Asp Asp Val Leu Ile Ala Asp Ser Ser Thr Lys Leu Thr
        195                 200                 205

Phe Glu Asp Ile Asp Ala Tyr Lys Ala Lys Val Phe Gln Trp Ala Ser
    210                 215                 220

Ala Asn Ala Ala Asp Pro Ser Val His Phe Trp Ile Ala Gly Glu Thr
225                 230                 235                 240

Ser Met Val Arg Phe Ile Arg Lys Glu Leu Ile Asn Ser Tyr Arg Val
                245                 250                 255

Asp Ser Ser Arg Ile Thr Phe Leu Gly Tyr Trp Lys Tyr Gly Arg Arg
            260                 265                 270

Thr Val Asp
        275

<210>  2
<211>  828
<212>  DNA
<213>  Corynebacterium glutamicum 0807

<400>  2
atgtccacag ctctccccga tcagctcaag tgggaataca gtgccttccc cgtgcagatc      60

tcgcagaagc aacggcttag tcccggcttc atgcggatca ccgtcactgg tgacaagctc     120

cgattctttg gccagtgggg tttggaccaa cgcatcaaac tgatcattcc aagcccggct     180

gggaacatcc cagatttcgg aattctcgac gaacccactc ccccaccgac aacgtggctt     240

cctcgtgcta agtcttttcc agcggaccaa cgaccgatct tgcgcaccta caccccatct     300

gcggtccgac ccgaactatg cgaagtagac attgatatct atcttcacaa cccttcggga     360

ccagtatcca gatgggcaaa gaactgcagt gttgacgatg aactaatcat caccggccct     420

gacgtacgcg caggagaaac cggctacgga atcacctatc atccgacttc tgcgatcgat     480

cgcctctgtc tcatcggcga ttgtgcatca gctcccgcga tcgcaaatat cgtcaatcaa     540

tcaaaagtac ctactacggt tttcctccac gtagacagcc tagaagatga tgtattgatc     600

gccgatagct ccaccaagct cactttcgaa gacatcgacg cttacaaagc aaaggtcttc     660

caatgggctt cagccaatgc agcagatcct tcagtacact tctggatcgc cggtgaaact     720

agcatggtgc gcttcattcg caaagaacta atcaacagct accgagttga ttcctcacga     780

atcactttcc tcggctactg gaaatacggc cgacgaaccg tagactag                    828

<210>  3
<211>  188
<212>  PRT
<213>  Corynebacterium glutamicum 0600

<400>  3

Met Ala Asp Thr Glu Arg Glu Leu Ala Asp Leu Val Pro Gln Ala Thr
1               5                   10                  15

Ala Gly Asp Arg Arg Ala Leu Gln Arg Ile Met Glu Ile Ile His Pro
            20                  25                  30

Ile Val Leu Arg Tyr Ala Arg Ala Arg Ile Gly Gly Gly Arg Gln Pro
        35                  40                  45

Thr Ala Glu Asp Val Ala Gln Glu Ile Cys Leu Ala Val Ala Thr Ser
    50                  55                  60

Ile Arg Asn Phe Val Asp Gln Gly Arg Pro Phe Met Ala Phe Val Tyr
65                  70                  75                  80

Gly Ile Ala Ser Asn Lys Val Ala Asp Ala His Arg Ala Met Ser Arg
                85                  90                  95

Asp Lys Ser Thr Pro Ile Glu Glu Val Pro Glu Thr Ser Pro Asp Thr
            100                 105                 110

Phe Thr Pro Glu Asp Phe Ala Leu Val Ser Asp Gly Ser Asn Arg Val
        115                 120                 125

Arg Glu Leu Leu Asp Leu Leu Ser Glu Lys Ala Arg Asp Ile Leu Ile
    130                 135                 140

Leu Arg Val Ile Val Gly Leu Ser Ala Glu Glu Thr Ala Glu Met Val
145                 150                 155                 160

Gly Ser Thr Pro Gly Ala Val Arg Val Ala Gln His Arg Ala Leu Thr
                165                 170                 175

Thr Leu Arg Ser Thr Leu Glu Gln Gln Glu Asn Lys
            180                 185

<210>  4
<211>  567
<212>  DNA
<213>  Corynebacterium glutamicum 0600

<400>  4
ttggctgata ctgagcgcga gctcgctgac ctggtaccgc aggcaacggc gggcgatcgt      60

cgggcattgc aaagaataat ggagattatt caccccattg ttttgcgtta tgctcgcgct 120

cgtattggag gtggacgcca gccaacggca gaagacgttg ctcaagaaat ctgcctggcg 180

gtagccacct ccattaggaa ctttgtcgac cagggtaggc cgttcatggc gtttgtctac 240

ggcattgcat ctaacaaggt cgcagatgct cacagggcga tgtcgaggga taaatcgact 300

cctattgagg aagtcccaga aacttcacca gatactttta cccccgaaga ctttgcgctg 360

gtcagcgatg gaagtaacag agttagggaa cttctcgatc tactgagtga aaaggcacgc 420

gacattctta tcttgagagt tatcgttggt ctttccgcag aagaaactgc agagatggtg 480

ggcagcaccc caggtgctgt acgagttgcc caacacaggg cactcacgac acttcgaagc 540

acacttgagc agcaggagaa caagtaa 567

<210> 5
<211> 230
<212> PRT
<213> Corynebacterium glutamicum 1427

<400> 5

Met Pro Ala Gly Gly Leu Ile Val Ala Ile Asp Gly Pro Ser Gly Thr
1               5                   10                  15

Gly Lys Ser Thr Thr Ser Arg Ala Leu Ala Thr Arg Leu Ser Ala Lys
            20                  25                  30

Tyr Leu Asp Thr Gly Ala Met Tyr Arg Val Ala Thr Leu His Val Leu
        35                  40                  45

Asn Gln Gly Ile Asp Pro Ala Asp Ser Ala Ala Val Ile Ala Ala Thr
    50                  55                  60

Ala Val Leu Pro Leu Ser Ile Ser Asp Asp Pro Ala Ser Thr Glu Val
65                  70                  75                  80

Leu Leu Ala Gly Val Asp Val Gln Lys Asp Ile Arg Gly Pro Glu Val
                85                  90                  95

Thr Gln Asn Val Ser Ala Val Ser Ala Ile Pro Glu Val Arg Glu Asn
            100                 105                 110

Leu Val Ala Leu Gln Arg Ala Leu Ala Ala Lys Ala His Arg Cys Val
        115                 120                 125

Val Glu Gly Arg Asp Ile Gly Thr Ala Val Leu Val Asp Ala Pro Ile
    130                 135                 140

Lys Ala Phe Leu Thr Ala Ser Ala Glu Val Arg Ala Gln Arg Arg Phe
145                 150                 155                 160

Asp Gln Asp Thr Ala Ala Gly Arg Asp Val Asp Phe Asp Ala Val Leu
                165                 170                 175

Ala Asp Val Val Arg Arg Asp Glu Leu Asp Ser Thr Arg Ala Ala Ser
            180                 185                 190

Pro Leu Lys Pro Ala Asp Asp Ala His Ile Val Asp Thr Ser Asp Met
        195                 200                 205

Thr Met Asp Gln Val Leu Asp His Leu Ile His Leu Val Glu Ala Ser
    210                 215                 220

Ala Glu Arg Ser Asn Gln
225                 230

<210>  6
<211>  693
<212>  DNA
<213>  Corynebacterium glutamicum 1427

<400>  6
atgcctgccg gtggcctcat cgtagccatc gacgggccgt ctggcaccgg aaaatccacc      60

acatcccgcg cgctcgcaac ccgtctctcg gccaagtacc tagatactgg tgcgatgtac     120

cgcgtcgcaa cgcttcatgt gcttaaccag gggattgacc ctgcagatag cgcagccgtg     180

atcgctgcaa ccgctgtatt gccgttgtcg atttctgacg atcccgcctc cactgaggtg     240

ttgctcgcgg gcgtcgatgt gcaaaaggac atccgcggac cagaagtcac ccaaaatgtc     300

tccgcagtgt ccgcgatccc tgaggttcgt gaaaacttgg tggcgttgca gcgcgcactc     360

gccgccaaag cacatcgctg cgtcgtcgaa ggcagagaca tcggaacggc agtgcttgtc     420

gacgcgccca tcaaggcgtt tctcaccgcc tcagcggaag tccgcgccca gcgacgcttt     480

gaccaagaca ccgcagcagg tcgcgacgta gatttcgacg ctgtgctggc agatgttgtt     540

cgccgcgatg aactagattc cacccgtgcc gcctcaccgc tgaaaccagc agatgatgca     600

cacatcgtgg acacctctga tatgaccatg gatcaagtac ttgatcacct catccaccta     660

gtggaagcct ccgctgaaag gagcaaccag tga                                  693

<210>  7
<211>  105
<212>  PRT
<213>  Corynebacterium glutamicum 1102

<400>  7

Met Thr Tyr Thr Ile Ala Gln Pro Cys Val Asp Val Leu Asp Arg Ala
1               5                   10                  15

Cys Val Glu Glu Cys Pro Val Asp Cys Ile Tyr Glu Gly Lys Arg Met

20                  25                  30

Leu Tyr Ile His Pro Asp Glu Cys Val Asp Cys Gly Ala Cys Glu Pro
        35                  40                  45

Ala Cys Pro Val Glu Ala Ile Phe Tyr Glu Asp Asp Val Pro Asp Glu
    50                  55                  60

Trp Leu Asp Tyr Asn Asp Ala Asn Ala Ala Phe Phe Asp Asp Leu Gly
65                  70                  75                  80

Ser Pro Gly Gly Ala Ala Lys Leu Gly Pro Gln Asp Phe Asp His Pro
                85                  90                  95

Met Ile Ala Ala Leu Pro Pro Gln Ala
            100                 105

<210>  8
<211>  318
<212>  DNA
<213>  Corynebacterium glutamicum 1102

<400>  8
atgacataca caatcgcaca gccctgcgtt gacgtcttgg atcgtgcctg cgttgaagaa      60

tgcccagtag attgcatcta cgaaggtaag cgcatgctgt acatccaccc ggatgagtgc     120

gttgactgtg gtgcatgtga gcctgcttgc ccagttgagg caatcttcta cgaggacgat     180

gtcccagacg aatggcttga ctacaacgat gccaacgctg cattcttcga tgatctgggc     240

tccccaggtg gtgcggctaa gcttggacca caagattttg atcacccaat gatcgctgcg     300

ctgccgcctc aggcataa                                                   318

<210>  9
<211>  474
<212>  PRT
<213>  Corynebacterium glutamicum 2859

<400>  9

Met Thr Pro Thr Arg Arg Ile Leu Leu Trp Ala Trp Thr Thr Val Leu
1               5                   10                  15

Leu Gly Ser Leu Leu Trp Pro Leu Ala Ala Pro Gly Glu Leu Leu Leu
            20                  25                  30

Arg Asp Met Ser Val Val Asp His Pro Ala Leu Ser Leu Asn Ala Leu
        35                  40                  45

Gly Phe Gly Asp Leu Pro Ser Arg Asn Ala Pro Gln Asp Gly Val Leu
    50                  55                  60

Ala Leu Leu Gly Phe Leu Pro Val Ser Trp Ile Val Arg Thr Met Leu
65                  70                  75                  80

Leu Val Ala Gly Phe Ala Gly Ala Trp Gly Ala Met Arg Leu Gly Pro
                85                  90                  95

Ser Lys Phe Leu Ala Val Thr Val Ala Ile Tyr Asn Pro Phe Val Val
            100                 105                 110

Glu Arg Leu Leu Gln Gly His Trp Ser Leu Val Met Ala Val Trp Leu
        115                 120                 125

Leu Pro Leu Val Val Ala Leu Arg Arg His Pro Arg Trp Gln Val Val
    130                 135                 140

Ala Ile Trp Ala Ala Ser Leu Thr Pro Thr Gly Ala Val Val Ala Ala
145                 150                 155                 160

Val Thr Gly Val Ala Ser Ser Lys Arg Lys Arg Phe Thr Thr Leu Cys
                165                 170                 175

Ser Phe Leu Ser Trp Leu Pro Trp Leu Ile Pro Ala Leu Leu Ala Thr
            180                 185                 190

Pro Thr Ser Gly Gly Ala Leu Thr Phe Ala Ile Arg Ser Glu Thr Tyr
        195                 200                 205

Ala Gly Thr Leu Gly Thr Ala Leu Gly Leu Gly Gly Ile Trp Asn Ala
    210                 215                 220

Gly Ala Val Pro Ala Ser Arg Glu Leu Gly Phe Ala Val Ala Gly Ile
225                 230                 235                 240

Leu Leu Phe Ala Ile Leu Leu Ala Gly Phe Lys Asn Cys Pro Trp Val
                245                 250                 255

Leu Ala Leu Leu Ala Val Val Gly Phe Met Gly Ala Ile Gly Pro Trp
            260                 265                 270

Leu Met Pro Asn Leu Phe Thr Trp Thr Ile Ala Tyr Val Pro Gly Ala
        275                 280                 285

Ala Leu Phe Arg Asp Ser Gln Lys Leu Leu Met Leu Ala Ile Pro Ala
    290                 295                 300

Tyr Val Cys Leu Ala Ala Gly Val Lys Ser Pro Leu Ser Trp Val Ala
305                 310                 315                 320

Thr Gly Leu Ala Leu Leu Gln Ile Pro Asp Ala Pro Arg Glu Val Ser

325                 330                 335

Val Ile Arg Pro Ser Ser Ala His Val Glu Ser Val Glu Ala Leu Ala
            340                 345                 350

Glu Ile Ala Asp Gly Arg Asp Ile Leu Ile Ile Gly Gln Gly Pro Leu
        355                 360                 365

Val Thr Arg Glu Asp Gly Ile Pro Val Val Asp Pro Lys Thr Lys Ala
    370                 375                 380

Leu Ser Val Val Glu Ser Gly Glu Leu Arg Val Asp Gly Ile Ile Thr
385                 390                 395                 400

Asp Ala Pro Ser Gln Arg Trp Thr Glu Ala Thr Gln Ala Trp Ala Ala
                405                 410                 415

Gly Asp Ile Glu Arg Leu Glu Glu Leu Gly Val Gly Val Val Val Asp
            420                 425                 430

Gly Asp Thr Ile Thr Glu Thr Gly Ala Pro Pro Gln His Gly Trp Lys
        435                 440                 445

Tyr Tyr Leu Gly Val Gly Leu Thr Val Leu Trp Met Ala Leu Pro Leu
    450                 455                 460

Gly Leu Leu Phe Arg Arg Lys Thr Lys Lys
465                 470

<210>  10
<211>  1425
<212>  DNA
<213>  Corynebacterium glutamicum 2859

<400>  10
atgaccccga cccgccgtat cctgctgtgg gcatggacga ctgtgctttt gggttctttg      60

ctgtggccgt tggctgcgcc tggcgagttg ttgttgcggg atatgtcggt ggtggaccat     120

cctgcgttgt cgttgaatgc gttgggtttt ggtgatttgc cgtcgcggaa cgccccgcag     180

gatggtgtgc tggcgttgct tggctttttg ccggtgagtt ggatcgttcg aaccatgctg     240

cttgtggcgg gtttcgcggg cgcgtggggt gcgatgcgcc ttgggccttc caagtttttg     300

gccgttaccg tggcgattta caatcccttc gtggtggagc gtctgctgca gggccattgg     360

tcgttggtga tggcggtgtg gctgttgccg ctggttgtgg cgctgcgcag gcatccgcgt     420

tggcaggttg tggcgatctg ggcggcgtcg ttgacgccca cgggtgcggt ggttgcggcg     480

gtcacgggcg tggcgagttc taaaagaaaa cgctttacga cgctatgttc cttcctttcg     540

tggcttcctt ggctaatacc tgcgcttctt gccaccccca cttcgggagg tgcgctgacc     600

ttcgccattc gttctgaaac atatgcagga acgttgggaa ctgcgctggg cctgggtgga 660

atttggaacg cgggggccgt gccggcctca cgcgaactgg gtttcgcggt tgctggaatt 720

ttgttatttg cgattctgct ggcgggtttc aaaaactgtc cgtgggttct cgcactgctg 780

gcggtcgtag gttttatggg ggcgatcggt ccgtggctga tgccgaatct gttcacgtgg 840

accatcgcat atgttccagg cgccgcgctg tttagggatt ctcaaaaact cctcatgctg 900

gctatccctg cctatgtgtg tttggccgcc ggggtgaaaa gcccactgtc gtgggtggct 960

accggtttgg cgttgctcca gattcctgat gcaccacgtg aggtttccgt gatacgccca 1020

agttcagcgc atgtggaatc agtggaagca ctggcagaaa tcgctgatgg ccgcgacatc 1080

ttaatcatcg gccaaggccc cttggtgacc cgcgaggatg ggatcccggt tgtcgatccc 1140

aaaaccaaag ccctctccgt ggtggaatcc ggcgaactgc gtgtggacgg aatcatcacc 1200

gacgcgccct cacagcggtg gaccgaagca acgcaggcat gggcggccgg ggatatcgag 1260

cgccttgaag aacttggcgt tggtgtcgtg gtggatggag atacgatcac agaaactggc 1320

gcaccaccgc agcatggctg gaaatactac ctcggtgtgg gcctgaccgt gctgtggatg 1380

gcgttgccgc tgggactact ttttcgacgc aagaccaaga agtag 1425

<210> 11
<211> 301
<212> PRT
<213> Corynebacterium glutamicum 2857

<400> 11

Met Asn Pro Arg Trp Arg Met Gly Ala Tyr Asp Trp Val Asp Ile Ile
1               5                   10                  15

Ser Thr Cys Glu Phe Ser Gly Lys Val Trp Ala Val Phe Met Lys Arg
            20                  25                  30

Ser Ala Thr Val Leu Ile Ile Ala Gly Val Leu Phe Leu Ile Phe Ala
        35                  40                  45

Phe Thr Val Pro Pro Tyr Val Thr Gly Gln Ala Arg Thr Ile Pro Lys
    50                  55                  60

Asp Leu Asp Leu Thr Leu Val Ser Glu Ser Pro Gln Gly Phe Val Arg
65                  70                  75                  80

Thr Glu His Ile Val Thr Ala Pro Thr Glu Lys Val Asp Glu Ile Ala
                85                  90                  95

Thr His Val Asp Gln Thr Val Thr Asp Val Gln Gly Lys Thr Val Ala
            100                 105                 110

Glu Ile Ser Asp Asp Val Val Leu Ile Gly His Ser Arg Tyr Pro Val

115                 120                 125

Ile Lys Pro Thr Ala Thr Ile Ser Gly Ser Pro Ala Asp Ser Ser Asn
    130                 135                 140

Val Val Arg Glu Gly Leu His Tyr Phe Phe Pro Ala Asn Thr Leu Arg
145                 150                 155                 160

Asn Ser Tyr Pro Tyr Tyr Asp Ile Val Leu Gly Glu Asp Ser Pro Val
                165                 170                 175

Asp Tyr Val Ser Arg Glu Gly Asn Thr Tyr Thr Phe Tyr Gln His Leu
            180                 185                 190

Arg Tyr Val Pro Leu Asp Asp Ser His Thr Tyr Ser Val Glu Arg Thr
        195                 200                 205

Leu Lys Val Asp Arg Phe Ser Gly Ile Ile Val Ala Lys Asp Glu Ala
    210                 215                 220

Met Thr Phe His Gly Pro Asp Gly Asp Asp Thr Val Glu Phe Thr Tyr
225                 230                 235                 240

Thr Ala Asp Thr Leu Lys Leu Leu Gln Asp His Ala His Asp Ile Asp
                245                 250                 255

Gln Arg Leu Ser Trp Ala Lys Gly Phe Asp Phe Phe Ser Lys Phe Leu
            260                 265                 270

Gly Leu Ser Leu Leu Ala Ile Gly Val Phe Leu Thr Gly Ile Phe Lys
        275                 280                 285

Arg Gly Gln Leu Met Ser Thr Val Asn Lys Leu Arg Ser
    290                 295                 300

<210>  12
<211>  906
<212>  DNA
<213>  Corynebacterium glutamicum 2857

<400>  12
atgaatccgc gatggcggat gggtgcatat gattgggtag acattatttc aacatgcgag      60

tttagcggaa aggtgtgggc tgtttttatg aagcgatctg caacggtcct cattattgcg     120

ggcgtgctgt tcctcatttt tgccttcacg gtaccgccgt atgtgactgg tcaggcgcgg     180

acgattccga aggatttgga tctgacgttg gtgagcgaaa gtccgcaggg gtttgtgcgc     240

actgaacata ttgtgactgc tccgacggaa aaggtcgatg agatcgcgac gcatgtggat     300

cagacagtta cggatgtgca ggggaaaact gttgcggaaa tttcggatga tgtggtgttg     360

attggacact ctcgttatcc ggtgattaag ccgactgcca ccatttcggg ttcgccggcg 420

gatagtagca atgtggtgcg ggaggggttg cattacttct tcccggctaa tacgttgcgg 480

aattcttatc cctattatga catcgtattg ggtgaggatt ccccggtgga ttatgtctcg 540

cgcgagggca atacttatac cttctaccag catcttcgtt atgttccatt ggatgattct 600

cacacctatt cggtggagcg gaccctgaaa gtggatcgtt tttccggcat cattgtggct 660

aaagatgagg cgatgacgtt tcatggccca gacggcgatg acacagtaga attcacttat 720

actgcggata cgttgaagct tctgcaggat catgcgcatg atattgatca gcggttgtcg 780

tgggctaagg ggtttgattt cttttctaaa ttcttaggcc tgtcgttgct tgcgattggt 840

gtgttcctca cgggaatttt caagcgcggc cagctgatga gcactgtgaa taaactcagg 900

agttaa 906

<210> 13
<211> 334
<212> PRT
<213> Corynebacterium glutamicum 2525

<400> 13

Met Ala Ala Asp Ser Asp Leu Ser Val His Asp Ala Tyr Leu Lys Glu
1               5                   10                  15

His Val Ala Pro Val Lys Ala Ile Asn Trp Asn Ser Ile Pro Asp Ser
            20                  25                  30

Lys Asp Leu Glu Val Trp Asp Arg Leu Thr Gly Asn Phe Trp Leu Pro
        35                  40                  45

Glu Lys Val Pro Val Ser Asn Asp Ile Lys Ser Trp Gly Thr Leu Asn
    50                  55                  60

Glu Val Glu Lys Ala Ala Thr Met Arg Val Phe Thr Gly Leu Thr Leu
65                  70                  75                  80

Leu Asp Thr Ile Gln Gly Thr Val Gly Ala Ile Ser Leu Leu Pro Asp
                85                  90                  95

Ala Asp Ser Leu His Glu Glu Ala Val Leu Thr Asn Ile Ala Phe Met
            100                 105                 110

Glu Ser Val His Ala Lys Ser Tyr Ser Asn Ile Phe Met Thr Leu Ala
        115                 120                 125

Ser Thr Ala Glu Ile Asn Asp Ala Phe Arg Trp Ser Glu Glu Asn Glu
    130                 135                 140

Asn Leu Gln Arg Lys Ala Lys Ile Ile Leu Ser Tyr Tyr Glu Gly Asp

145                 150                 155                 160

Asp Pro Leu Lys Arg Lys Ile Ala Ser Val Ile Leu Glu Ser Phe Leu
                165                 170                 175

Phe Tyr Ser Gly Phe Tyr Leu Pro Met Tyr Trp Ser Ser His Ser Lys
            180                 185                 190

Leu Thr Asn Thr Ala Asp Val Ile Arg Leu Ile Ile Arg Asp Glu Ala
        195                 200                 205

Val His Gly Tyr Tyr Ile Gly Tyr Lys Tyr Gln Lys Ala Val Ala Lys
    210                 215                 220

Glu Thr Pro Glu Arg Gln Glu Glu Leu Lys Glu Tyr Thr Phe Asp Leu
225                 230                 235                 240

Leu Tyr Asp Leu Tyr Asp Asn Glu Thr Gln Tyr Ser Glu Asp Leu Tyr
                245                 250                 255

Asp Asp Leu Gly Trp Thr Glu Asp Val Lys Arg Phe Leu Arg Tyr Asn
            260                 265                 270

Ala Asn Lys Ala Leu Asn Asn Leu Gly Tyr Glu Gly Leu Phe Pro Ala
        275                 280                 285

Asp Glu Thr Lys Val Ser Pro Asn Ile Leu Ser Ala Leu Ser Pro Asn
    290                 295                 300

Ala Asp Glu Asn His Asp Phe Phe Ser Gly Ser Gly Ser Ser Tyr Val
305                 310                 315                 320

Ile Gly Lys Ala Glu Asn Thr Glu Asp Asp Asp Trp Asp Phe
                325                 330

<210>  14
<211>  1005
<212>  DNA
<213>  Corynebacterium glutamicum 2525

<400>  14
atggctgctg attctgatct cagtgttcac gatgcttact taaaggagca tgttgcacct      60

gtaaaggcga tcaactggaa ctccatccca gattccaaag atcttgaagt ctgggatcgt     120

ctgaccggta acttctggct cccagaaaag gtcccagtat ccaacgacat caagagctgg     180

ggaaccctca acgaggttga aaaagccgca accatgcgcg tgttcaccgg acttaccctg     240

ctggacacca ttcagggcac tgtcggcgca atctccctgc ttccagacgc agattcactg     300

cacgaagaag cggtgctaac caacattgcg ttcatggaat ccgtgcacgc aaagagttac     360

tccaacatct tcatgactct ggcctccacc gcggaaatca acgatgcgtt ccgttggtct 420

gaggaaaatg aaaacctgca gcgcaaggca aagatcatcc tgtcttacta tgagggcgat 480

gatccactaa agcgcaagat cgcctccgtg atcctggagt ccttcctgtt ctactccggc 540

ttctacctcc caatgtattg gtccagccac tccaagctga ccaacaccgc cgacgtgatc 600

cgcctgatca tccgcgatga ggcagtgcac ggctactaca ttggctacaa gtatcaaaag 660

gctgtcgcga aggagactcc agagcgtcag gaagagctga aggagtacac cttcgatctg 720

ctctacgatc tttacgataa cgaaactcag tactccgaag atctctacga cgatcttgga 780

tggaccgagg atgttaagcg attccttcgc tacaacgcca acaaggccct caacaacctt 840

ggctacgaag gactcttccc agcggatgaa accaaggtgt ccccaaacat cttgtctgcg 900

ctgtcaccaa acgctgatga gaaccacgac ttcttctccg gctccggttc ctcttacgtt 960

attggtaagg cagaaaacac cgaggatgat gactgggact tctaa 1005

<210> 15
<211> 707
<212> PRT
<213> Corynebacterium glutamicum 2530

<400> 15

Met Pro Arg Gly Asp Gln Met Asp Phe His Ala Leu Asn Ala Leu Leu
1               5                   10                  15

Asn Leu Tyr Asp Asp Asn Gly Lys Ile Gln Phe Glu Lys Asp Arg Glu
            20                  25                  30

Ala Ala Asn Gln Tyr Phe Leu Gln His Val Asn Gln Asn Thr Val Phe
        35                  40                  45

Phe His Asn Leu Gln Glu Lys Ile Asp Tyr Leu Val Glu Asn Lys Tyr
    50                  55                  60

Tyr Asp Pro Ile Val Leu Asp Lys Tyr Asp Phe Gln Phe Ile Lys Asp
65                  70                  75                  80

Leu Phe Lys Arg Ala Tyr Gly Phe Lys Phe Arg Phe Gln Ser Phe Leu
                85                  90                  95

Gly Ala Tyr Lys Tyr Tyr Thr Ser Tyr Thr Leu Lys Thr Phe Asp Gly
            100                 105                 110

Arg Arg Tyr Leu Glu Arg Phe Glu Asp Arg Val Cys Met Val Ala Leu
        115                 120                 125

Thr Leu Ala Asp Gly Asp Arg Ala Leu Ala Glu Asn Leu Val Asp Glu
    130                 135                 140

Ile Met Ser Gly Arg Phe Gln Pro Ala Thr Pro Thr Phe Leu Asn Ser
145                 150                 155                 160

Gly Lys Ala Gln Arg Gly Glu Pro Val Ser Cys Phe Leu Leu Arg Ile
                165                 170                 175

Glu Asp Asn Met Glu Ser Ile Gly Arg Ser Ile Asn Ser Ala Leu Gln
            180                 185                 190

Leu Ser Lys Arg Gly Gly Gly Val Ala Leu Leu Leu Ser Asn Leu Arg
        195                 200                 205

Glu Ala Gly Ala Pro Ile Lys Lys Ile Glu Asn Gln Ser Ser Gly Val
    210                 215                 220

Ile Pro Val Met Lys Leu Leu Glu Asp Ala Phe Ser Tyr Ala Asn Gln
225                 230                 235                 240

Leu Gly Ala Arg Gln Gly Ala Gly Ala Val Tyr Leu Asn Ala His His
                245                 250                 255

Pro Asp Ile Leu Ser Phe Leu Asp Thr Lys Arg Glu Asn Ala Asp Glu
            260                 265                 270

Lys Ile Arg Ile Lys Thr Leu Ser Leu Gly Val Val Ile Pro Asp Ile
        275                 280                 285

Thr Phe Glu Leu Ala Lys Arg Asn Asp Asp Met Tyr Leu Phe Ser Pro
    290                 295                 300

Tyr Asp Val Glu Arg Ile Tyr Gly Lys Pro Phe Ala Asp Val Ser Ile
305                 310                 315                 320

Thr Glu His Tyr Asp Glu Met Val Asp Asp Asp Arg Ile Arg Lys Thr
                325                 330                 335

Lys Ile Asn Ala Arg Gln Phe Phe Gln Thr Leu Ala Glu Ile Gln Phe
            340                 345                 350

Glu Ser Gly Tyr Pro Tyr Ile Met Tyr Glu Asp Thr Val Asn Ala Ser
        355                 360                 365

Asn Pro Ile Glu Gly Arg Ile Thr His Ser Asn Leu Cys Ser Glu Ile
    370                 375                 380

Leu Gln Val Ser Thr Pro Ser Glu Phe Asn Asp Asp Leu Thr Tyr Ala
385                 390                 395                 400

Glu Val Gly Glu Asp Ile Ser Cys Asn Leu Gly Ser Leu Asn Val Ala
                405                 410                 415

Met Ala Met Asp Ser Pro Asn Phe Glu Lys Thr Ile Glu Thr Ala Ile
            420                 425                 430

Arg Gly Leu Thr Ala Val Ser Glu Gln Thr Ser Ile Asp Ser Val Pro
        435                 440                 445

Ser Ile Arg Lys Gly Asn Glu Ala Ala His Ala Ile Gly Leu Gly Gln
    450                 455                 460

Met Asn Leu His Gly Tyr Phe Gly Arg Glu His Met His Tyr Gly Ser
465                 470                 475                 480

Glu Glu Ala Leu Asp Phe Thr Asn Ala Tyr Phe Ala Ala Val Leu Tyr
                485                 490                 495

Gln Cys Leu Arg Ala Ser Asn Lys Ile Ala Thr Glu Arg Gly Glu Arg
            500                 505                 510

Phe Lys Asn Phe Glu Asn Ser Lys Tyr Ala Thr Gly Glu Tyr Phe Asp
        515                 520                 525

Asp Phe Asp Ala Asn Asp Phe Ala Pro Lys Ser Asp Lys Val Lys Glu
    530                 535                 540

Leu Phe Ala Lys Ser Asn Ile His Thr Pro Thr Val Glu Asp Trp Ala
545                 550                 555                 560

Ala Leu Lys Ala Asp Val Met Glu His Gly Leu Phe Asn Arg Asn Leu
                565                 570                 575

Gln Ala Val Pro Pro Thr Gly Ser Ile Ser Tyr Ile Asn Asn Ser Thr
            580                 585                 590

Ser Ser Ile His Pro Ile Ala Ser Lys Ile Glu Ile Arg Lys Glu Gly
        595                 600                 605

Lys Ile Gly Arg Val Tyr Tyr Pro Ala Pro His Met Asp Asn Asp Asn
    610                 615                 620

Leu Glu Tyr Phe Glu Asp Ala Tyr Glu Ile Gly Tyr Glu Lys Ile Ile
625                 630                 635                 640

Asp Thr Tyr Ala Val Ala Thr Lys Tyr Val Asp Gln Gly Leu Ser Leu
                645                 650                 655

Thr Leu Phe Phe Lys Asp Thr Ala Thr Thr Arg Asp Ile Asn Arg Ala

660                 665                 670

Gln Ile Tyr Ala Trp Arg Lys Gly Ile Lys Thr Leu Tyr Tyr Ile Arg
        675                 680                 685

Leu Arg Gln Val Ala Leu Glu Gly Thr Glu Val Asp Gly Cys Val Ser
    690                 695                 700

Cys Met Leu
705

<210>  16
<211>  2124
<212>  DNA
<213>  Corynebacterium glutamicum 2530

<400>  16
gtgccacgcg gagaccagat ggacttccac gctcttaacg cgttgctcaa cctttacgat      60

gacaacggca agatccagtt tgagaaagac cgtgaagctg caaaccagta cttcctgcag     120

cacgtcaacc agaacaccgt cttcttccac aacctgcagg aaaagatcga ctacctggtt     180

gaaaacaagt actatgaccc aatcgttctg gacaagtacg acttccagtt catcaaggac     240

ctcttcaagc gcgcatacgg attcaagttc cgcttccagt ccttcctcgg tgcatacaag     300

tactacactt cctacaccct gaagaccttc gacggtcgcc gctacctcga gcgtttcgaa     360

gaccgtgtct gcatggtcgc cctcaccctc gctgacggcg accgcgcatt ggccgagaac     420

ctggtcgatg agatcatgtc tggccgtttc caaccagcaa ccccaacctt cctgaactcc     480

ggcaaggcac agcgcggcga gccagtatcc tgcttcctcc tgcgtatcga agacaacatg     540

gagtccatcg gacgttccat caactctgct cttcagctgt ccaagcgtgg cggtggcgta     600

gcgttgctgc tgtccaacct tcgtgaagcc ggtgcaccga ttaagaagat tgaaaaccag     660

tcttccggtg ttatcccagt gatgaaactt ctggaagatg ctttctccta cgctaaccag     720

ctgggtgctc gtcagggtgc aggtgctgtg tacctcaacg ctcaccaccc agatatcctg     780

tccttcctgg ataccaagcg tgagaacgcc gatgagaaga tccgcatcaa gaccctgtcc     840

ctgggtgttg tgattccgga catcaccttc gagctggcta agcgcaacga tgacatgtac     900

ctgttctccc catacgatgt ggagcgcatt tacggcaagc ctttcgcaga cgtctcaatc     960

accgagcact acgacgagat ggtggatgat gaccgcatcc gcaagaccaa gatcaacgcg    1020

cgtcagttct tccagaccct ggcagaaatc cagttcgagt ccggttaccc atacatcatg    1080

tatgaagaca ccgtgaatgc atccaaccca atcgaaggtc gcatcaccca ctcaaacctg    1140

tgctctgaga tccttcaggt gtccacccca tctgaattca acgatgacct gacttacgca    1200

gaggtcggcg aagacatttc ttgtaacttg ggttccctca acgttgcaat ggctatggat    1260

tcaccaaact ttgagaagac catcgaaacc gcaatccgcg gcttaactgc agtgtctgag    1320

cagaccagca tcgattccgt gccttccatc cgtaagggca acgaagcagc tcacgccatc 1380

ggccttggcc agatgaacct tcacggctac ttcggtcgcg agcacatgca ctacggctcc 1440

gaggaagccc tggacttcac caacgcatac tttgctgccg tgctgtacca gtgcctgcgt 1500

gcatccaaca agatcgctac tgagcgtgga gagcgtttca agaacttcga aaactccaag 1560

tatgcaaccg gtgagtactt cgatgatttc gatgcaaacg acttcgcacc aaagtccgac 1620

aaggtcaagg aactctttgc caagtcgaac atccacaccc caaccgttga ggactgggct 1680

gcgctgaagg ccgacgtgat ggagcacggt ctgttcaacc gtaacctgca agcggttcca 1740

ccaaccggtt cgatctccta catcaacaac tccacctcgt cgatccaccc aatcgcatcc 1800

aagattgaga tccgcaagga aggcaagatc ggccgcgttt actacccagc tccacacatg 1860

gacaatgaca accttgagta cttcgaggac gcctacgaaa tcggctacga gaagatcatt 1920

gacacctacg ctgtggcaac caagtacgtt gaccagggcc tgtcactgac cttgttcttc 1980

aaggacactg ccaccacccg tgacatcaac cgtgcgcaga tctacgcatg gcgcaagggc 2040

atcaagacct tgtactacat tcgcctgcgc caggttgctc tggaaggcac tgaagttgac 2100

ggctgcgtca gctgcatgct gtaa 2124

<210> 17
<211> 148
<212> PRT
<213> Corynebacterium glutamicum 2531

<400> 17

Met Leu Ile Val Tyr Phe Ser Ser Ala Thr Asp Asn Thr His Arg Phe
1               5                   10                  15

Val Gln Lys Leu Asp Leu Pro Asn Val Arg Ile Pro Leu Thr Arg Val
            20                  25                  30

Glu Glu Pro Leu Lys Ile Asn Glu Pro Tyr Val Leu Ile Thr Pro Thr
        35                  40                  45

Tyr Gly Gly Gly Val Ser Met Thr Gly Glu Asn Ser Arg Pro Val Pro
    50                  55                  60

Pro Gln Val Ile Arg Phe Leu Asn Asp Glu His Asn Arg Ser Phe Ile
65                  70                  75                  80

Arg Ala Val Val Ala Gly Gly Asn Ser Asn Phe Gly Ser Asp Phe Gly
                85                  90                  95

Leu Ala Gly Glu Ile Ile Ser Lys Lys Cys Lys Val Pro Tyr Val Tyr
            100                 105                 110

Arg Phe Glu Leu Met Gly Asn Glu Glu Asp Val Ser Ile Leu Arg Gly

115                 120                 125

Gly Leu Thr Gln Asn Ala Gln Ala Leu Gly Leu Glu Pro Gln Glu Pro
    130                 135                 140

Val Thr Ser Arg
145

<210>  18
<211>  447
<212>  DNA
<213>  Corynebacterium glutamicum 2531

<400>  18
atgctaatcg tgtatttttc ctcggccacc gacaacacgc atcgttttgt acaaaagctc      60

gatttaccca acgtgcgcat ccccctcact agggtggaag aaccgctgaa aatcaacgag     120

ccctacgtgc taatcacccc gacctatggt ggtggagtct ccatgactgg agaaaactcc     180

cgcccggtcc caccacaagt catcaggttt ttaaatgatg aacacaaccg cagcttcatc     240

agggcagttg ttgcaggtgg aaactcaaac ttcggctccg attttgggtt ggcaggcgag     300

atcatttcca agaaatgtaa agtgccctat gtctaccgtt tcgagctcat gggcaatgag     360

gaagatgtaa gtatccttcg tggaggtctt actcaaaacg cccaagcttt ggggctggaa     420

ccacaagaac cagttacctc gcgataa                                         447

<210>  19
<211>  77
<212>  PRT
<213>  Corynebacterium glutamicum 2532

<400>  19

Met Ala Ile Thr Val Tyr Thr Lys Pro Ala Cys Val Gln Cys Asn Ala
1               5                   10                  15

Thr Lys Lys Ala Leu Asp Arg Ala Gly Leu Glu Tyr Asp Leu Val Asp
            20                  25                  30

Ile Ser Leu Asp Glu Glu Ala Arg Glu Tyr Val Leu Ala Leu Gly Tyr
        35                  40                  45

Leu Gln Ala Pro Val Val Val Ala Asp Gly Ser His Trp Ser Gly Phe
    50                  55                  60

Arg Pro Glu Arg Ile Arg Glu Met Ala Thr Ala Ala Ala
65                  70                  75

<210>  20
<211>  234
<212>  DNA
<213>  Corynebacterium glutamicum 2532

<400>  20
atggcaatca ccgtttacac caagccagct tgcgtccagt gcaatgccac caagaaggcc      60

ctcgaccgcg ctggtcttga gtatgacctc gttgatatca gccttgatga agaggcacgt     120

gagtacgtcc tcgcacttgg ctacctgcag gcaccagttg tcgttgcaga tggctcccac     180

tggtccggtt tccgcccaga gcgcatccgt gaaatggcaa ccgcagctgc ctaa           234


<210>  21
<211>  26
<212>  DNA
<213>  Artificial

<220>
<223>  Primer

<400>  21
ggtggatccg gtggaatttg gcctgc                                           26


<210>  22
<211>  23
<212>  DNA
<213>  Artificial

<220>
<223>  Primer

<400>  22
tcaggatccc atccgggtga gac                                              23


<210>  23
<211>  20
<212>  DNA
<213>  Artificial

<220>
<223>  Primer

<400>  23
gtaggatccc actggttgcc                                                  20


<210>  24
<211>  25
<212>  DNA
<213>  Artificial

<220>
<223>  Primer

<400>  24
gaaggatcca actcaccggc aaaaa                                            25


<210>  25
<211>  26
<212>  DNA
<213>  Artificial

<220>

<223>  Primer

<400>  25
ctgggtaccg gagtgttttg ggttgt                                            26


<210>  26
<211>  26
<212>  DNA
<213>  Artificial

<220>
<223>  Primer

<400>  26
cacggtacca gcggaattcg cggaaa                                            26


<210>  27
<211>  27
<212>  DNA
<213>  Artificial

<220>
<223>  Primer

<400>  27
atgggatcct agtggcgata atttagg                                           27


<210>  28
<211>  26
<212>  DNA
<213>  Artificial

<220>
<223>  Primer

<400>  28
cttggatcca aaggtgtgaa ggggtt                                            26